# FASCICULE DE BREVET EUROPEEN

(11) **EP 0 581 979 B1**
(45) Date de publication et mention de la délivrance du brevet: **03.03.1999**
(21) Numéro de dépôt: 92113189.2
(22) Date de dépôt: 03.08.1992
(51) Int. Cl.: C07D 307/00, C07C 67/29

(54) **Dérivés de l'acide quinique et procédé de préparation de dérivés de l'acide quinique**
Chinasäurederivate und Verfahren zur Herstellung von Chinasäurederivaten
Quinic acid derivatives and process to prepare quinic acid derivatives

(43) Date de publication de la demande: 09.02.1994
(73) Titulaire: SOCIETE DES PRODUITS NESTLE S.A., 1800 Vevey (CH)
(72) Inventeur: Huynh-Ba, Tuong, CH-1009 Pully (CH)
(74) Mandataire: Archambault, Jean

(56) Documents cités:
- WO-A-86/01508
- GB-A- 802 668
- CHEMICAL ABSTRACTS, vol. 116, no. 23, 8 Juin 1992, Columbus, Ohio, US; abstract no. 236095z, page 880 ;

## Description

L'invention concerne de nouveaux dérivés de l'acide quinique et un procédé de préparation de dérivés de l'acide quinique.

Les dérivés de l'acide quinique sont largement répandus dans le monde végétal. Le contenu en acide chlorogénique des fèves de café vert, par exemple, peut représenter jusque 10% de la matière sèche. Le terme "acide chlorogénique" recouvre en fait un mélange relativement complexe de dérivés de l'acide quinique parmi lesquels on peut citer les acides caféoyl-, dicaféoyl-, p-coumaroyl-, féruloyl- et caféoylféruloyl-quinique. Après torréfaction, les grains de café contiennent un mélange de composés encore plus complexe dont certains sont constitués de lactones des acides quiniques appelées quinides ou encore d'autres isomères formés lors du traitement thermique. Certains de ces dérivés possèdent une activité antimicrobienne, antagoniste des opiacés, antiinflammatoire, fongistatique, cytostatique et inhibitrice de la biosynthèse des leucotriènes. Nous avons trouvé que certains de ces composés possédaient une activité anti-uréase dont l'application fait l'objet de la demande de brevet parallèle de la titulaire déposée sous le titre "composition cosmétique ou dermatologique anti-uréase".

La synthèse des acides hydroxycinnamoyl-quiniques est décrite par exemple dans J. Chem. Soc. (London) 1964, 2137-2146, E. Haslam et col. Cependant, les procédés connus requièrent une étape de séparation délicate et laborieuse basée sur la technique de distribution à contre-courant en vue d'isoler le composé cherché dans la mesure où l'étape clé d'estérification n'est pas régiosélective et conduit à un mélange d'isomères qu'il faut séparer. De plus, la méthode connue fait usage de groupes protecteurs des réactifs hydroxycinnamiques qui ne peuvent être clivés que dans des conditions drastiques (acide fort ou base forte et chaleur) qui provoquent l'isomérisation des dérivés chlorogéniques.

Le brevet WO-A-86/01508 décrit la synthèse d'antagonistes des opiacés, en particulier des quinides 1, 3 ou 4 monosubstituées. Dans ce document, le choix des groupes de protection et des méthodes de clivage présente des inconvénients non négligeables. Les conditions de clivage, par un traitement avec K2CO3 dans du méthanol aqueux, sont telles que le risque d'ouverture partielle de la lactone et d'isomérisation du groupe féruloyl ou isoféruloyl de la position 1 et positions 3, 4 et 5 devient non négligeable, une purification ultérieure devient nécessaire, ce qui diminue d'autant le rendement de la réaction.

Le document GB-A-802668 décrit des esters caféiques de l'acide quinique et de la quinide, en particulier la 1-O-caféoyl quinide, sa préparation et la synthèse des acides 1-O-caféoyl et 1,4-O-dicaféoyl quiniques par hydrolyse des quinides correspondantes. Cette méthode de préparation est susceptible de provoquer des réactions d'isomérisation partielle du groupe caféoyl en positions 4 et 5, d'oxydation du groupe catéchol de l'acide quinique et de polymérisation. De plus, un tel procédé n'est pas sélectif, ce qui conduit inévitablement à un mélange de composés.

L'article CA 116, 23, 236095z, décrit la synthèse de l'acide 4,5 bis-(3,4-diméthoxycinnamoyl) quinique. Le choix dans les conditions de préparation dudit acide quinique à partir de la quinide correspondante a l'inconvénient de présenter un risque d'isomérisation du groupe 3,4-diméthoxycinnamoyl des positions 4 et 5 en positions 1 et 3 du squelette d'acide quinique qui n'est pas négligeable.

L'invention concerne des quinides destinés à servir de composés intermédiares dans la synthèse d'acides quiniques 1 ou 5 monosubstitués ou 3,4-bis-substitué correspondant à la formule générale: 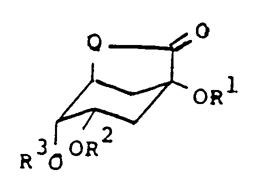 dans laquelle R1 représente H et R2 et R3 ont la même signification et représentent un groupe 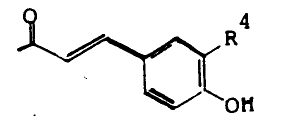 et R4 représente H, OCH3 ou OH ou R1 représente un groupe p-coumaroyl et R2 et R3 représentent H.

Les nouveaux composés selon l'invention constituent des produits intermédiaires très utiles dans la préparation des acides quiniques ayant les applications intéressantes mentionnées précédemment.

L'invention concerne également un procédé de préparation de dérivés 1 ou 5 monosubstitués ou 3,4-bis-substitués de l'acide quinique, dans lequel on fait réagir un dérivé d'un acide hydroxycinnamique dont le ou les groupes hydroxyles sont protégés, avec un dérivé de l'acide quinique de manière à former un ester, puis on clive le ou les groupes protecteurs.
Ce procédé est caractérisé par le fait que, dans le cas des dérivés 1 monosubstitués et 3,4-bis-substitués, le dérivé de l'acide quinique mis en oeuvre est une quinide et que le dérivé d'acide quinique final est obtenu par hydrolyse acide de la quinide dans des conditions ménagées, que l'on protège les fonctions OH en position 3,4 et 5 de l'acide quinique par réaction avec l'acétone conduisant à la 3,4-O-isopropylidène quinide, que le ou les groupes protecteurs des groupes hydroxyles de l'acide hydroxycinnamique sont des carbonates, et que l'on clive séquentiellement le groupe protecteur acétonide, puis le ou les groupes protecteurs carbonates dans des conditions ménagées d'acidité et de température, les dites conditions ménagées permettant de réaliser une régiospécificité de l'estérification et d'éviter toute dégradation ou isomérisation lors de la déprotection.

Dans un premier mode de réalisation du procédé selon l'invention, on prépare avec un bon rendement des dérivés 1-O-hydroxycinnamoyl de la quinide que l'on transforme ensuite en l'acide quinique.
Pour ce faire, on protège les fonctions OH en position 3, 4 et 5 de l'acide quinique par exemple par réaction avec l'acétone en présence d'acide paratoluènesulfonique, ce qui conduit à la 3,4-O-isopropylidène-quinide.
Le goupe hydroxyl en position 1 se trouve alors seul disponible pour être estérifié par un dérivé réactif d'un acide hydroxycinnamique dont la ou les fonctions phénol ont été préalablement protégées, un tel dérivé réactif pouvant par exemple être un anhydride ou de préférence un chlorure d'acide.
Le groupe protecteur de la fonction phénol choisi est un carbonate, par exemple le trichloroéthyl carbonate dans le cas de l'acide p-coumarique et de l'acide férulique, qui peut être clivé dans des conditions modérées, par exemple par le zinc en milieu acétique à la température ambiante. Dans le cas de l'acide caféique, on préfère utiliser le méthyl carbonate que l'on clive facilement dans des conditions modérées, par exemple par le chlorure de lithium en présence de trichlorométhylsilane dans la pyridine à l'ébullition, sans qu'il ne se produise d'isomérisation décelable.
Avant le clivage du carbonate protecteur de l'acide hydroxycinnamique, il est nécessaire de libérer le groupe acétonide protecteur des fonctions OH en position 3 et 4 de la quinide. Pour ce faire, on utilise une solution aqueuse très concentrée d'acide trifluoroacétique à la température ambiante, ce qui permet d'éviter toute décomposition qui interviendrait dans les conditions classiques d'utilisation de l'acide acétique à haute température.
Enfin, pour préparer les acides quiniques correspondant aux quinides, on procède à l'hydrolyse acide de ces dernières, également dans des conditions ménagées, par exemple par l'acide chlorhydrique concentré dans un solvant polaire, par exemple le tétrahydrofurane à basse température, de préférence voisine de 0°C.

Selon un second mode de réalisation du procédé de l'invention de préparation de dérivés 3,4-bis-substitués, on utilise comme produit de départ la 3,4-O-isopropylidène quinide précédente dont on protège la position 1. Le groupe protecteur utilisé est de préférence le trichloroéthyl carbonate.

Les fonctions OH en position 3 et 4 sont ensuite rendues accessibles par clivage du groupe acétonide protecteur comme indiqué précédemment. On fait alors réagir la quinide déprotégée avec un dérivé de l'acide hydroxycinnamique dont la ou les fonctions phénol ont été préalablement protégées, un tel dérivé réactif pouvant être par exemple un anhydride ou de préférence un chlorure d'acide. Comme indiqué précédemment en liaison avec le premier mode de réalisation, on utilise de préférence des groupes protecteurs tricloroéthylcarbonate pour préparer les dérivés p-coumaroyl et féruloyl et méthylcarbonate pour les dérivés caféoyl.
On procède ensuite au clivage des groupes protecteurs, puis à la rupture du cycle lactone de la quinide comme indiqué précédemment.

Dans un troisième mode de réalisation du procédé de l'invention destiné à préparer les acides quiniques 5-hydroxycinnamoyl substitués, on part d'une quinide 3,4-O protégée, par exemple par un groupe acétonide dont on ouvre ensuite le cycle lactone de manière à libérer la fonction OH en position 5 en vue de son estérification après avoir protégé le groupe carboxyl. Pour ce faire, on utilise de préférence un halogénure de phénacyl , par exemple le bromure de phénacyl qui permet l'estérification de la fonction carboxyl en position 1, donc sa protection par un groupe labile, après avoir ouvert le cycle lactone par le bicarbonate de sodium. Le phénacylester mis en oeuvre peut être clivé dans des conditions ménagées, par exemple par le zinc dans l'acide acétique à la température ambiante.
On estérifie ensuite la fonction OH en position 5 par un dérivé réactif d'un acide hydroxycinnamique dont la ou les fonctions phénol ont été préalablement protégées, par exemple pour les acides p-coumarique et férulique par le trichloroéthyl carbonate et pour l'acide caféique par le méthyl carbonate ou le benzylidène. Après l'estérification, on procède au clivage séquentiel des différents groupes protecteurs en commençant par libérer les groupes des substituants périphériques, par exemple les groupes phénacyl protecteur de la fonction carboxyl en position 1 et carbonate, puis le cas échéant le groupe benzylidène protecteurs des fonctions phénol des acides hydroxycinnamiques et le groupe acétonide protecteur des fonctions OH en position 3 et 4 du squelette de l'acide quinique. Le groupe benzylidène peut être clivé aisément par l'acide trifluoroacétique en présence d'eau à la température ambiante. Quant aux autres groupes protecteurs, ils peuvent également être clivés dans les conditions modérées indiquées précédemment.

Les composés préparés dans ces différents modes de réalisation sont obtenus avec un très bon rendement car chaque étape conduit à une préparation sans isomérisation et les composés peuvent ensuite être purifiés facilement.

Les exemples ci-après illustrent l'invention.
Dans ceux-ci, les parties et pourcentages sont pondéraux sauf indication contraire.
Les composés de départ et les réactifs proviennent de Fluka AG. Les solvants ont été préalablement rendu anhydres avant usage. On a réalisé la chromatographie liquide moyenne pression (MPLC) sur un équipement Büchi.
Le polyamide mis en oeuvre dans la chromatographie a été préalablement lavé au méthanol, puis séché avant utilisation.
La structure des composés a été vérifiée par résonance magnétique nucléaire du proton (RMN). Les résultats de l'analyse élémentaire, les rendements et les points de fusion sont indiqués.

### Exemples 1-3

### 1. Synthèse de la 1-O-p-coumaroyl quinide et de l'acide 1-O-p-coumaroyl quinique

### 1.1. 3,4-O-Isopropylidène quinide

On traite au reflux une suspension de 66,2 g (344,4 mmole) d'acide quinique et de 2,1 g (11 mmole) d'acide p-toluènesulfonique dans 900 ml d'acétone pendant 24 h dans un appareil de Soxhlet contenant 90 g de tamis moléculaire de 4 A°. Après refroidissement à O°C, on ajoute 27 g (321,4 mmole) de bicarbonate de sodium. Après agitation du mélange réactionnel 1 h à la température de 0°C, on filtre la suspension et on évapore le solvant. Une recristallisation du résidu dans un mélange dichlorométhane/hexane conduit à 67 g de 3,4-O-isopropylidène quinide de point de fusion (p.f.) 141-142 °C avec un rendement (rdt) de 91%.

### 1.2. 1-O-Carbotrichloroéthoxy-p-coumaroyl-3,4-O-isopropylidène quinide

A une solution de 3,2 g (15 mmole) de 3,4-O-isopropylidène quinide et 1,42 g (18 mmole) de pyridine dans 80 ml de dichlorométhane, on ajoute 5,7 g (16 mmole) de chlorure d'acide carbotrichloroéthoxy- p-coumarique à O°C. Après agitation 24 h à la température ambiante, on élimine les solvants par évaporation. On reprend alors le résidu par l'acétate d'éthyl , on le rince avec une solution aqueuse 0,5 N d' HCl, puis de saumure, on sèche la solution sur sulfate de sodium et on évapore le solvant. Une recristallisation du résidu dans l'éthanol conduit à 6,58 g de 1-O-carbotrichloroéthoxy-p-coumaroyl-3,4-O-isopropylidène quinide (p.f. 178-182°C, rdt 82%).

### 1.3. 1-O-Carbotrichloroéthoxy-p-coumaroyl quinide

On agite une solution de 5,36 g (10 mmole) de 1-O-Carbotrichloroéthoxy-p-coumaroyl-3,4-O-isopropylidène quinide dans 22 ml d'une solution aqueuse à 90% d'acide trifluoroacétique pendant 2 h à la température ambiante. Après évaporation des solvants sous le vide de la trompe à eau, puis trituration du résidu dans 30 ml d'éther on obtient 3,71 g de 1-O-carbotrichloroéthoxy-p-coumaroyl quinide (p.f. 221-224°C, rdt 75%).

### 1.4. 1-O-p-Coumaroyl quinide

On ajoute 1,9 g (30 mmole) de poudre de zinc à une solution de 2,76 g (5,7 mmole) de 1-O-carbotrichloroéthoxy-p-coumaroyl quinide dans 30 ml de dioxanne. Après 3 h d'agitation à la température ambiante, on elimine les solvants par évaporation. On reprend ensuite le résidu dans 50 ml d'acétate d'éthyl, on rince la solution avec une solution aqueuse 0,5 N d'HCl, puis de saumure, on décante et on sèche la phase organique par le sulfate de sodium. Après évaporation du solvant et recristallisation du résidu dans l'acétonitrile, on obtient 1,39 g de 1-O-p-coumaroyl quinide (p.f. 212-216°C, rdt 78%).
Analyse élémentaire: calculé, C=60,00; H=5,04, trouvé, C=59,87; H=5,12.

### 1.5. Acide 1-O-p-coumaroyl quinique

On ajoute 15 ml d'HCl 5N à une solution de 2,8 g (8,74 mmole) de 1-O-p-coumaroyl quinide dans 15 ml de tétrahydrofuranne (THF) à 0°C. Après agitation 24 h à la température ambiante, on dilue le mélange réactionnel avec 20 ml de THF, puis on ajoute par portions du bicarbonate de sodium solide tout en maintenant le pH à 3. Après saturation de la solution avec le NaCl, on recueille la phase aqueuse et on l'extrait avec 3x30 ml de THF. On sèche ensuite les phases organiques combinées, puis on évapore le solvant. Après précipitation du résidu par un mélange chloroforme/THF suivie d'une lyophilisation, on obtient 2,09 g d'acide 1-O-p-Coumaroyl quinique (rdt 71%).
Analyse élémentaire: calculé, C=56,80; H=5,36, trouvé, C=56,54; H=5,54.

### 2. Synthèse de la 1-O-féruloyl quinide et de l'acide 1-O-féruloyl quinique

En procédant comme indiqué aux paragraphes 1.2-1.5 précédents, on obtient successivement
**2.1, la 1-O-carbotrichloroéthoxyféruloyl-3,4-O-isopropylidène quinide** (p.f. 159-161,5°C, rdt 76%),
**2.2, la 1-O-carbotrichloroéthoxyféruloyl quinide** (p.f. 176-178,5°C, rdt 73%),
**2.3, la 1-O-féruloyl quinide** (p.f. 205-207,5°C, rdt 85%), analyse élémentaire: calculé, C=58,29; H=5,18, trouvé, C=58,08; H=5,37 et
**2.4, l'acide 1-O-féruloyl quinique** (rdt 72%), analyse élémentaire: calculé, C=55,43; H=5,47, trouvé, C=55,12; H=5,35.

### 3. Synthèse de la 1-O-caféoyl quinide et de l'acide 1-O-caféoyl quinique

En procédant de manière analogue à ce qui est indiqué aux paragraphes 1.2-1.3 et 1.5 précédents, mais en utilisant le méthyl carbonate comme groupe protecteur des fonctions phénol de l'acide caféique, on obtient successivement
**3.1, la 1-O-dicarbométhoxycafeoy1-3,4-O-isopropylidène quinide,** la purification finale de la quinide ayant lieu par chromatographie du résidu sur colonne de polyamide en éluant avec un mélange de 1 volume de dichlorométhane pour 3 volumes d'hexane, (rdt 57%),
**3.2, la 1-O-dicarbométhoxycaféoyl quinide** (rdt 62%),
**3.3, la 1-O-caféoyl quinide** (p.f. 225-229 avec décomposition, rdt 75%), analyse élémentaire: calculé, C=57,14; H=4,80, trouvé, C=57,11; H=4,91, puis
**3.4, l'acide 1-O-caféoyl quinique** (rdt 72%), analyse élémentaire: calculé, C=54,24; H=5,12, trouvé, C=53,97; H=5,21.
Dans l'étape 3.3, le clivage des groupes protecteurs diffère de l'étape 1.4 précédente et a lieu de la manière suivante:
On traite à reflux une solution de 5,5 g (12,16 mmole) de 1-O-dicarbométhoxycaféoyl quinide et de 5,43 g (50 mmole) de chlorotriméthylsilane dans 50 ml de pyridine pendant 1 h. On ajoute ensuite 5,3 g (122 mmole) de LiCl et on continue le traitement sous reflux pendant 1 h. Après élimination de la pyridine par évaporation, on reprend le résidu par 100 ml d'acétate d'éthyl, on rince avec une solution aqueuse d'HCl 1N, puis de saumure et on sèche la phase organique sur sulfate de sodium. Après réduction du volume de la solution à 30 ml, on ajoute 70 ml d'hexane et enfin on recristallise le résidu solide par l'acétate d'éthyl.

### Exemples 4-5

### 4. Synthèse de l'acide 5-O-féruloyl quinique

### 4.1. Phénacyl 3,4-O-isopropylidène quinate

On traite au reflux une suspension de 1,07 g (5 mmole) de 3,4-O-isopropylidène quinide dans 5 ml d'une solution aqueuse 1M de bicarbonate de sodium pendant 1 h. Après élimination de l'eau sous vide, on triture le résidu dans un mélange équivolumique d'éthanol et de toluène, puis on le concentre jusqu'à dessiccation. On répète 3 fois cette trituration et ce séchage. On traite le solide blanc résultant avec 1 g (5 mmole) de bromure de phénacyl dans 10 ml de diméthylformamide pendant 1 h.
Après évaporation du solvant sous vide, on dissout le résidu dans 20 ml de dichlorométhane, on lave à l'eau, sèche sur sulfate de sodium et évapore le solvant. La recristallisation du résidu dans l'acétate d'éthyl conduit à 1,46 g de Phénacyl 3,4-O-isopropylidène quinate (p.f. 136-139°C, rdt 84%).

### 4.2. Phénacyl 5-O-carbotrichloroéthoxyféruloyl-3,4-O-isopropylidène quinate

On ajoute à une solution de 3,88 g (10 mmole) de chlorure d'acide carbotrichloroéthoxyférulique dans 10 ml de dichlorométhane une solution de 3,5 g (10 mmole) de phénacyl 3,4-O-isopropylidène quinate et de 0,87 g (11mmole) de pyridine dans 30 ml de dichlorométhane à -10°C. Après agitation 24 h à la température ambiante, on lave le mélange réactionnel avec une solution aqueuse 0,5N de HCl, on décante, sèche la phase organique sur sulfate de sodium et évapore le solvant. On chromatographie le résidu sur une colonne de silicagel que l'on élue avec le dichlorométhane, puis on traite le produit avec un mélange de 1 volume de benzène pour 4 volumes d'hexane, ce qui conduit à un résidu amorphe de 2,84 g de phénacyl 5-O-carbotrichloroéthoxyféruloyl-3,4-O-isopropylidène quinate (rdt 41%).

### 4.3. Acide 5-O-féruloyl-3,4-O-isopropylidène quinique

On traite une solution de 0,2 g (0,29 mmole) de 5-O-carbotrichloroéthoxyféruloyl-3,4-O-isopropylidène quinate dans 2 ml d'acide acétique glacial avec 0,5 g (7,65 mmole) de poudre de zinc pendant 30 min, puis on y ajoute 15 ml d'hexane. Après élimination des solvants par filtration, on ajoute 10 ml de dichlorométhane au résidu, puis on le traite à O°C par une solution aqueuse d'HCl 1N, on sépare la phase organique et on extrait ensuite la phase aqueuse avec 3x10 ml de dichlorométhane. On lave les phases organiques combinées à la saumure, on les sèche sur sulfate de sodium et on en élimine le solvant par évaporation. Une reprise du résidu par un mélange équivolumique de dichlorométhane/hexane donne 0,07 g d'acide 5-O-féruloyl-3,4-O-isopropylidène quinique (rdt 62%).

### 4.4. Acide 5-O-féruloyl quinique

On agite une solution de 0,06 g (0,15 mmole) d'acide 5-O-féruloyl-3,4-O-isopropylidène quinique dans 4 ml d'une solution aqueuse à 90% d'acide trifluoroacétique pendant 30 min. Après évaporation des solvants sous vide, puis traitement du résidu avec un mélange de 1 volume d'acétate d'éthyl et 4 volumes d'hexane, on obtient 0,03 g d'acide 5-O-féruloyl quinique (rdt 54%).

### 5. Synthèse de l'acide 5-O-caféoyl quinique

### 5.1. 3,4-Benzylidènedioxy benzaldéhyde

On chauffe à 100°C sous agitation pendant 24 h un mélange de 13,8 g (100 mmole) de 3,4-dihydroxybenzaldéhyde, de 19,3 g (120 mmole) de chlorure de benzylidène et de 16,6 g (120 mmole) de carbonate de potassium dans 50 ml de diméthylformamide. Après évaporation du solvant sous vide, on ajoute 200 ml d'éther au résidu, on le lave avec de la saumure, on le sèche sur sulfate de sodium et on évapore l'éther. Une recristallisation du résidu dans l'hexane conduit à 13,5 g de 3,4-Benzylidènedioxy benzaldéhyde (p.f. 82-84°C, rdt 60%).

### 5.2. Acide 3,4-O-benzylidène caféique

On traite au reflux une solution de 3,4 g (15 mmole) de 3,4-benzylidènedioxy benzaldéhyde et de 1,19 g (11,43 mmole) d'acide malonique dans 15 ml de pyridine et 0,5 ml de pipéridine pendant 1 h. Après évaporation des solvants, on traite le résidu avec 8 ml d'HCl concentré dans 50g de glace, puis on l'extrait avec 3x50 ml d'un mélange de 9 volumes d'éthyl acétate pour 1 volume de THF. On lave ensuite les extraits combinés à la saumure, on les sèche sur sulfate de sodium et on élimine le solvant par évaporation. Un lavage du résidu au toluène, puis une recristallisation dans l'acétate d'éthyl donne 2,53 g d'acide 3,4-O-benzylidène caféique (p.f. 185-188°C, rdt 82%).

### 5.3. Phénacyl 5-O-benzylidène caféoyl-3,4-O-isopropylidène quinate

On ajoute à 0°C 0,72 g (3,6 mmole) de dicyclohexyl carbodiimide à une suspension de 1,05 g (3mmole) de phénacyl 3,4-O-isopropylidène quinate, de 0,8 g (3mmole) d'acide 3,4-O-benzylidène caféique et de 0,44 g (0,3 mmole) de 4-pyrrolidinopyridine dans 14 ml de dichlorométhane, puis on laisse réagir le mélange 4 h à la température ambiante. On lave ensuite le mélange réactionnel successivement avec 2x5 ml d'une solution aqueuse 0,1N d'HCl, puis 2x5 ml de saumure, on le sèche sur sulfate de sodium et on élimine les solvants par évaporation. On chromatographie le résidu sur une colonne de silicagel en éluant avec du dichlorométhane et on recueille 0,8 g de phénacyl 5-O-benzylidène caféoyl-3,4-O-isopropylidène quinate (rdt 45%).

### 5.4. Acide 5-O-benzylidène caféoyl-3,4-O-isopropylidène quinique

En procédant comme indiqué au paragraphe 4.3 ci-dessus à celà près que l'on traite le résidu de la première étape avec l'acétate d'éthyl au lieu du dichlorométhane et avec une solution aqueuse d'HCl 2N au lieu d'une solution de concentration 1N, on obtient l'acide 5-O-benzylidène caféoyl-3,4-O-isopropylidène quinique (rdt 88%).

### 5.5. Acide 5-O-caféoyl quinique

En procédant comme indiqué au paragraphe 4.4 ci-dessus, mais en recristallisant le produit dans de l'eau, on obtient l'acide 5-O-caféoyl quinique (rdt 86%), analyse élémentaire: calculé, C=54,24; H=5,12, trouvé, C=54,03; H=4,97.

### Exemples 6-8

### 6. Synthèse de la 3,4-O-di-p-coumaroyl quinide et de l'acide 3,4-O-di-p-coumaroyl quinique

### 6.1. 1-O-Carbotrichloroéthoxy-3,4-O-isopropylidène quinide

A une solution de 32,1 g (150 mmole) de 3,4-O-isopropylidène quinide et de 28,9 g (365 mmole) de pyridine dans 300 ml de dichlorométhane on ajoute goutte à goutte 34,96 g (165 mmole) de trichloroéthyl chloroformate dilué dans 30 ml de dichlorométhane à 0°C.
Après 3 h d'agitation à la température ambiante, on lave le mélange réactionnel avec une solution aqueuse d'HCl 2N, puis à l'eau, on sèche la solution sur sulfate de sodium et on évapore les solvants. Une recristallisation du résidu avec l'éthanol conduit à 49,5 g de 1-O-carbotrichloroéthoxy-3,4-O-isopropylidène quinide (p.f. 165-166°C, rdt 85%).

### 6.2. 1-O-Carbotrichloroéthoxy quinide

On agite une solution de 39 g (100 mmole) de 1-O-carbotrichloroéthoxy-3,4-O-isopropylidène quinide dans 60 ml d'une solution aqueuse à 90% d'acide trifluoroacétique pendant 3 h. Après élimination des solvants, on dissout le résidu dans 100 ml d'acétate d'éthyl, on le lave avec une solution aqueuse à 2% de bicarbonate de sodium, puis de la saumure, on le sèche sur sulfate de sodium et on évapore le solvant. Une recristallisation du résidu dans le toluène donne 24,6 g de 1-O-Carbotrichloroéthoxy quinide (p.f. 130-131°C, rdt 70%).

### 6.3. 3,4-O-Bis-carbotrichloroéthoxy-p-coumaroyl-1-O-carbotrichloroéthoxy quinide

On ajoute 7,88 g (22 mmole) de chlorure d'acide carbotrichloroéthoxy-p-coumarique à une solution de 3,5 g (10 mmole) de 1-O-Carbotrichloroéthoxy quinide et de 1,74 g (22 mmole) de pyridine dans 100 ml de dichlorométhane. Après agitation pendant 24 h, on lave le mélange réactionnel avec une solution aqueuse d'HCl 0,5N, puis à l'eau, on le sèche sur sulfate de sodium et on élimine les solvants par évaporation. La recristallisation du résidu dans un mélange acétate d'éthyl/hexane conduit à 5,3 g de 3,4-O-bis-carbotrichloroéthoxy-p-coumaroyl-1-O-carbotrichloroéthoxy quinide (p.f. 214-218°C, rdt 54%).

### 6.4. 3,4-O-Di-p-coumaroyl quinide

On traite 13,4 g (13,5 mmole) de 3,4-O-bis-carbotrichloroéthoxy-p-coumaroyl-1-O-carbotrichloroéthoxy quinide dans 200 ml de THF et 75 ml d'acide acétique avec 14 g (215 mmole) de poudre de zinc pendant 90 min à la température ambiante. Après élimination du résidu, on évapore le solvant du filtrat. On ajoute alors 200 ml d'acétate d'éthyl au résidu, on lave avec une solution aqueuse d'HCl 0,5N, puis à la saumure, on sèche sur sulfate de sodium et on évapore le solvant. Une recristallisation du produit brut avec l'acétate d'éthyl conduit à 4,33 g de 3,4-O-di-p-coumaroyl quinide (p.f. 233-235°C, rdt 69%), analyse élémentaire: calculé, C=64,38; H=4,75, trouvé, C=64,58; H=4,98.

### 6.5. Acide 3,4-O-di-p-coumaroyl quinique

On traite au reflux une solution de 2,8 g (6 mmole) de 3,4-O-di-p-coumaroyl quinide et de 0,1 ml d'acide trifluoroacétique dans 60 ml de THF aqueux à 80% pendant 3 j. Après élimination des solvants on ajoute 30 ml d'acétate d'éthyl au résidu, on lave la solution à la saumure, on la sèche sur sulfate de sodium et on en évapore le solvant. Une recristallisation du résidu dans un mélange de 1 volume d'acétate d'éthyl pour 5 volumes de dichlorométhane donne 1,94 g d'acide 3,4-O-di-p-coumaroyl quinique (rdt 67%), analyse élémentaire: calculé, C=61,98; H=4,99, trouvé, C=61,90; H=5,11.

### 7. Synthèse de la 3,4-diféruloyl quinide et de l'acide 3,4-O-diféruloyl quinique

En procédant d'une manière analogue à celle indiquée aux paragraphes 6.3-6.5 précédents, on obtient successivement:
- **7.1.**: **La 3,4-O-bis-carbotrichloroéthoxyféruloyl-1-0-carbotrichloroéthoxy quinide** (p.f. 234-235°C, rdt 67%), mise à part la recristallisation finale du résidu dans un mélange chloroforme/hexane,
- **7.2.**: **La 3,4-O-diféruloyl quinide** (rdt 77%), analyse élémentaire: calculé, C=61,60; H=4,98, trouvé, C=61,82; H=5,24, mise à part la recristallisation finale du produit brut dans le toluène,
- **7.3.**: **L'acide 3,4-O-diféruloyl quinique** (rdt 65%), analyse élémentaire: calculé, C=59,56; H=5,18, trouvé, C=59,30; H=5,16.

### 8. Synthèse de la 3,4-O-dicaféoyl quinide et de l'acide 3,4-O-dicaféoyl quinique

### 8.1. 3,4-O-Bis-dicarbométhoxycaféoyl-1-0-carbotrichloroéthoxy quinide.

A une solution de 17,9 g (50 mmole) de 1-0-carbotrichloroéthoxy quinide et de 7,9 g (100 mmole) de pyridine dans 150 ml de dichlorométane on ajoute à 0°C 31,4 g (100 mmole) de chlorure d'acide dicarbométhoxy caféique. Après 2 j. d'agitation à -10°C, on lave le mélange réactionnel avec une solution aqueuse d'HCl 1N, puis à l'eau, on le sèche sur sulfate de sodium et on en évapore les solvants. Une MPLC du résidu sur polyamide en éluant avec un mélange de 1 volume de dichlorométhane pour 4 volumes d'hexane conduit à 23,7 g de 3,4-O-bis-dicarbométhoxycaféoyl-1-O-carbotrichloroéthoxy quinide (rdt 52%).

### 8.2. 3,4-O-Dicaféoyl quinide

On traite au reflux 5,8 g (6,4 mmole) de 3,4-O-bis-dicarbométhoxycaféoyl-1-0-carbotrichloroéthoxy quinide et 2,8 g (64 mmole) de LiCl dans 60 ml de pyridine pendant 2 h. Après élimination du solvant, on ajoute 200 ml d'acétate d'éthyl au résidu, on lave la solution avec une solution aqueuse d'HCl 1N, puis à la saumure, on sèche sur sulfate de sodium et on évapore le solvant. Une MPLC du résidu sur polyamide en éluant avec un mélange de 6 volumes d'acétate d'éthyl pour 1 volume de méthanol, suivie d'une lyophilisation donne 2,38 g de 3,4-0-dicaféoyl quinide (rdt 75%), analyse élémentaire: calculé, C=60,24; H=4,45, trouvé, C=60,07; H=4,62.

### 8.3 Acide 3,4-O-dicaféoyl quinique

On traite au reflux une solution de 2 g (4 mmole) de 3,4-O-dicaféoyl quinide et de 20 gouttes d'acide trifluoroacétique dans 20 ml d'acétonitrile aqueux à 50% pendant 24 h. Après évaporation des solvants, on dissout le résidu dans 200 ml d'eau et on le lave avec 3x20 ml d'éther. Après décantation, on recueille la phase aqueuse et on la lyophilise, ce qui donne 1,25 g d'acide 3,4-O-dicaféoyl quinique (rdt 61%), analyse élémentaire. Calculé, C=58,14; H=4,68, trouvé, C=58,34; H=4,92.

Les dérivés 1-0-féruloyl quinide et 1-0-caféoyl quinide respectivement préparés selon les exemples 2 et 3 ne font plus partie de l'objet des revendications.

## Revendications

1. Quinides, destinés à servir de composés intermédiaires dans la synthèse d'acides quiniques 1 ou 5 monosubstitués ou 3,4-bis-substitués, de formule 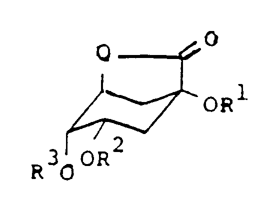 dans laquelle R1 représente H et R2 et R3 ont la même signification et représentent un groupe 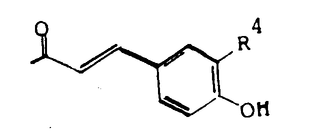 et R4 représente H, OCH3 ou OH ou R1 représente un groupe p-coumaroyl et R2 et R3 représentent H.

2. Procédé de préparation de dérivés 1 ou 5 monosubstitués ou 3,4-bis-substitués, dans lequel on fait réagir un dérivé d'un acide hydroxycinnamique dont le ou les groupes hydroxyles sont protégés, avec un dérivé de l'acide quinique de manière à former un ester, puis on clive le ou les groupes protecteurs caractérisé par le fait que, dans le cas des dérivés 1 monosubstitués et 3,4-bis-substitués, le dérivé de l'acide quinique mis en oeuvre est une quinide et que le dérivé d'acide quinique final est obtenu par hydrolyse acide de la quinide dans des conditions ménagées, que l'on protège les fonctions OH en position 3,4 et 5 de l'acide quinique par réaction avec l'acétone conduisant à la 3,4-O-isopropylidène quinide, que le ou les groupes protecteurs des groupes hydroxyles de l'acide hydroxycinnamique sont des carbonates, et que l'on clive séquentiellement le groupe protecteur acétonide, puis le ou les groupes protecteurs carbonates dans des conditions ménagées d'acidité et de température, les dites conditions ménagées permettant de réaliser une régiospécificité de l'estérification et d'éviter toute dégradation ou isomérisation lors de la déprotection.

3. Procédé selon la revendication 2, de préparation de dérivés 1-O-p-coumaroyl ou 1-O-féruoyl, caractérisé par le fait que le groupe protecteur de la fonction phénol est le trichloroéthyl carbonate, que l'on libère le groupe acétonide protecteur des positions 3 et 4 par l'acide trifluoroacétique aqueux à la température ambiante, puis que l'on clive le trichloroéthyl carbonate par le zinc en milieu acétique à la température ambiante.

4. Procédé selon la revendication 2, de préparation de dérivés 1-O-p-caféoyl, caractérisé par le fait que le groupe protecteur des fonctions phénol est le méthyl carbonate, que l'on libère le groupe acétonide protecteur des positions 3 et 4 par l'acide trifluoroacétique aqueux à la température ambiante, puis que l'on clive le méthyl carbonate par le chlorure de lithium en présence de trichlorométhylsilane dans la pyridine.

5. Procédé selon la revendication 2, de préparation de dérivés 3,4-bis-substitués, caractérisé par le fait que l'on fait réagir l'acide quinique avec l'acétone, que l'on protège la position 1 de la 3,4-isopropylidène quinide par le trichloroéthyl carbonate, que l'on clive le groupe acétonide protecteur des positions 3 et 4, puis que l'on fait réagir la quinide déprotégée avec un dérivé de l'acide hydroxycinnamique dont la ou les fonctions phénols sont protégées par un groupe carbonate et on clive les groupes protecteurs carbonates.

6. Procédé selon la revendication 2, de préparation d'un acide 5-0-hydroxycinnamoyl quinique, caractérisé par le fait que l'on fait réagir l'acide quinique avec l'acétone, que l'on ouvre le cycle lactone de la 3,4-isopropylidène quinide par réaction avec un halogénure de phénacyl de manière à protéger par un groupe phénylester le groupe carboxyl libéré en position 1, puis que l'on estérifie la fonction OH en position 5 par un dérivé réactif d'un acide hydroxycinnamique dont la ou les fonctions phénol sont protégées, pour les acides p-coumarique et férulique par le trichloroéthyl carbonate et pour l'acide caféique par le méthyl carbonate ou le benzylidène, puis que l'on clive successivement les groupes protecteurs phénacyl et carbonate, puis le cas échéant benzylidène.

7. Procédé selon l'une des revendications 2 à 6, caractérisé par le fait que l'on transforme la quinide en acide quinique par hydrolyse acide ménagée par l'acide chlorhydrique concentré à base température en présence d'un solvant polaire.

## Claims

1. Quinides, intended to act as intermediate compounds in the synthesis of 1 or 5 monosubstituted or 3,4-bis-substituted quinic acids, having the formula : 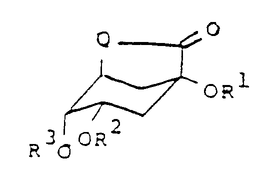 in which R1 represents H, and R2 and R3 have the same significance and represent a 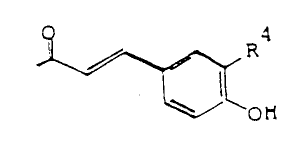 group, and R4 represents H, OCH₃ or OH, or R1 represents a p-coumaroyl group and R2 and R3 represent H.

2. Method for preparing 1 or 5 monosubstituted or 3,4-bis-substituted derivatives, in which a hydroxycinnamic acid derivative, of which the hydroxyl group or groups is/are protected, is made to react with a quinic acid derivative so as to form an ester, and the protective group or groups is/are then split off, characterized in that, in the case of 1 monosubstituted and 3,4-bis-substituted derivatives, the quinic acid derivative used is a quinide and the final quinic acid derivative is obtained by acid hydrolysis of the quinide under controlled conditions, that the OH functional groups in the 3, 4 and 5 positions of quinic acid are protected by reaction with acetone leading to 3,4-O-isopropylidene quinide, that the group or groups protecting the hydroxyl groups of hydroxycinnamic acid is/are carbonates, and that the acetonide protective group, followed by the carbonate protective group or groups are split off sequentially under controlled acidity and temperature conditions, the said controlled conditions making it possible to achieve regiospecificity of the esterification and to prevent any degradation or isomerisation during deprotection.

3. Method according to claim 2 for preparing 1-O-p-coumaroyl or 1-O-feruloyl derivatives, characterized in that the group protecting the phenol functional group is trichloroethyl carbonate, that the acetonide group protecting the 3 and 4 positions is liberated by aqueous trifluoroacetic acid at room temperature, and the trichloroethyl carbonate is then split off by zinc in an acetic medium at room temperature.

4. Method according to claim 2 for preparing 1-O-p-cafeoyl derivatives, characterized in that the group protecting the phenol functional groups is methyl carbonate, that the acetonide group protecting the 3 and 4 positions is liberated by aqueous trifluoroacetic acid at room temperature, and the methyl carbonate is then split off by lithium chloride in the presence of trichloromethylsilane in pyridine.

5. Method according to claim 2 for preparing 3,4-bis-substituted derivatives, characterized in that quinic acid is made to react with acetone, that the 1 position of 3,4-isopropylidene quinide is protected with trichloroethyl carbonate, that the acetonide group protecting the 3 and 4 positions is split off, and the deprotected quinide is then made to react with a hydroxycinnamic acid derivative of which the phenol functional group or groups is/are protected by a carbonate group and the protective carbonate groups are split off.

6. Method according to claim 2 for preparing a 5-O-hydroxycinnamoyl quinic acid, characterized in that quinic acid is made to react with acetone, that the lactone ring of the 3,4-isopropylidene quinide is opened by reaction with a phenacyl halide so as to protect with a phenylester group the carboxyl group liberated in the 1 position, the OH functional group in the 5 position is then esterified with a reactive derivative of a hydroxycinnamic acid of which the phenol functional group or groups is/are protected, for p-coumaric and ferulic acids by trichloroethyl carbonate and for cafeic acid by methyl carbonate or benzylidene, and the phenacyl and carbonate groups are then split off successively, followed, as the case may be, by benzylidene.

7. Method according to one of claims 2 to 6, characterized in that the quinide is converted into quinic acid by controlled acid hydrolysis with concentrated hydrochloric acid at a low temperature in the presence of a polar solvent.

## Patentansprüche

1. Chinide, die bestimmt sind, als Zwischenverbindungen bei der Synthese von 1- oder 5-monosubstitutierten oder 3,4-disubstituierten Chinasäuren zu dienen, der Formel 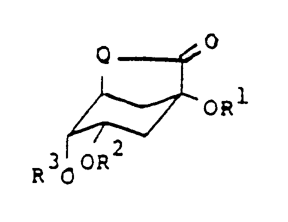 bei der R¹ für H steht und R² und R³ die gleiche Bedeutung aufweisen und für eine Gruppe 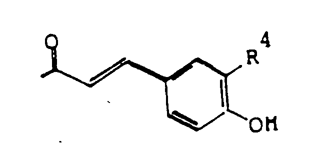 stehen und R⁴ für H, OCH₃ oder OH steht oder R¹ für eine p-Cumaroylgruppe steht und R² und R³ für H stehen.

2. Verfahren zur Herstellung von 1- oder 5-monosubstituierten oder 3,4-disubstituierten Derivaten, bei dem man ein Derivat einer Hydroxyzimtsäure, deren Hydroxylgruppe(n) geschützt sind, mit einem Chinasäurederivat so umsetzt, daß ein Ester gebildet wird, wonach man die Schutzgruppe(n) abspaltet, dadurch gekennzeichnet, daß im Falle von 1-monosubstitutierten und 3,4-disubstituierten Derivaten das eingesetzte Chinasäurederivat ein Chinid ist, und daß man das endgültige Chinasäurederivat durch saure Hydrolyse des Chinids bei schonenden Bedingungen gewinnt, daß man die OH-Funktionen in Position 3, 4 und 5 der Chinasäure durch Reaktion mit Aceton schützt, die zu einem 3,4-O-Isopropylidenchinid führt, daß die Schutzgruppe(n) der Hydroxylgruppen der Hydroxyzimtsäure Carbonate sind, und daß man nacheinander die Acetonidschutzgruppe, danach die Carbonatschutzgruppe(n) bei schonenden Säure- und Temperaturbedingungen abspaltet, wobei es die genannten schonenden Bedingungen gestatten, eine Regiospezifität der Veresterung zu erreichen und jeglichen Abbau oder Isomerisierung bei der Abspaltung der Schutzgruppen zu vermeiden.

3. Verfahren nach Anspruch 2, zur Herstellung von 1-O-p-Cumaroyl- oder 1-O-Feruloyl-Derivaten, dadurch gekennzeichnet, daß die Schutzgruppe der phenolischen Funktion Trichlorethylcarbonat ist, daß man die Acetonidschutzgruppe der Positionen 3 und 4 mit wässriger Trifluoressigsäure bei Umgebungstemperatur abspaltet, und daß man danach das Trichlorethylcarbonat mit Zink im essigsauren Milieu bei Umgebungstemperatur abspaltet.

4. Verfahren nach Anspruch 2, zur Herstellung von 1-O-p-Caffeoyl-Derivaten, dadurch gekennzeichnet, daß die Schutzgruppe der phenolischen Funktionen Methylcarbonat ist, daß man die Acetonidschutzgruppe der Positionen 3 und 4 mit wässriger Trifluoressigsäure bei Umgebungstemperatur abspaltet, und daß man danach das Methylcarbonat mit Lithiumchlorid in Gegenwart von Trichlormethylsilan in Pyridin abspaltet.

5. Verfahren nach Anspruch 2, zur Herstellung von 3,4-disubstituierten Derivaten, dadurch gekennzeichnet, daß man Chinasäure mit Aceton umsetzt, daß man die Position 1 des 3,4-Isopropylidenchinids mit Trichlorethylcarbonat schützt, daß man die Acetonidschutzgruppe der Positionen 3 und 4 abspaltet, wonach man das von der Schutzgruppe befreite Chinid mit einem Hydroxyzimtsäurederivat umsetzt, dessen phenolische Funktion(en) mit einer Carbonatgruppe geschützt sind, und daß man die Carbonatschutzgruppen abspaltet.

6. Verfahren nach Anspruch 2, zur Herstellung einer 5-O-Hydroxycinnamoylchinasäure, dadurch gekennzeichnet, daß man Chinasäure mit Aceton umsetzt, daß man den Lactonring des 3,4-Isopropylidenchinids durch Umsetzung mit einem Phenacylhalogenid auf eine solche Weise öffnet, daß eine Phenylestergruppe die in Position 1 freigesetzte Carboxylgruppe schützt, wonach man die OH-Funktion in Position 5 mit einem reaktiven Hydroxyzimtsäurederivat, dessen phenolische Funktion(en) geschützt sind, verestert, und zwar für die p-Cumarin- und Ferulasäure mit Trichlorethylcarbonat und für Kaffeesäure mit Methylcarbonat oder Benzyliden, wonach man nacheinander die Phenacyl- und Carbonatschutzgruppen abspaltet, danach gegebenenfalls Benzyliden.

7. Verfahren nach einem der Ansprüche 2 bis 6, dadurch gekennzeichnet, daß man das Chinid in Chinasäure durch schonende saure Hydrolyse mit konzentrierter Chlorwasserstoffsäure bei niedriger Temperatur in Gegenwart eines polaren Lösungsmittels umwandelt.
